(19)

Europäisches Patentamt

European Patent Office

Office européen des brevets

(11) **EP 4 245 129 A1**

(12) **EUROPEAN PATENT APPLICATION**
published in accordance with Art. 153(4) EPC

(43) Date of publication:
**20.09.2023 Bulletin 2023/38**

(21) Application number: **21891575.9**

(22) Date of filing: **13.10.2021**

(51) International Patent Classification (IPC):
**A01G 22/05** (2018.01)          **A01G 7/00** (2006.01)
**A01G 7/02** (2006.01)          **A01G 31/00** (2018.01)

(52) Cooperative Patent Classification (CPC):
**A01G 7/00; A01G 7/02; A01G 22/05; A01G 31/00**

(86) International application number:
**PCT/JP2021/037933**

(87) International publication number:
**WO 2022/102328 (19.05.2022 Gazette 2022/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.11.2020 JP 2020190528**

(71) Applicant: **FUJIFILM Corporation
Tokyo 106-8620 (JP)**

(72) Inventor: **HOSOKAWA, Takafumi
Ashigarakami-gun, Kanagawa 258-8577 (JP)**

(74) Representative: **Meissner Bolte Partnerschaft
mbB
Widenmayerstrasse 47
80538 München (DE)**

(54) **TOMATO PLANT, TOMATO FRUIT, AND METHOD FOR CULTIVATING TOMATO PLANT**

(57)     Provided is a tomato plant that satisfies Formula (1) and the application:

$$6.0 \leq L/X \leq 30.0 \ ... \ (1)$$

wherein in the formula, in a case where the tomato plant does not have a side branch, L represents a length in cm unit from a planting surface to a growth point of a main branch, and X represents a sum of the number of flower clusters and fruit clusters of the main branch, and in a case where the tomato plant has a side branch, L represents a length in cm unit from the planting surface to the growth point of one main branch or side branch, and X represents a sum of the number of flower clusters and fruit clusters of the one main branch or side branch, and where X is an integer of 2 or more.

FIG. 1

**Description**

**BACKGROUND OF THE INVENTION**

1. Field of the Invention

The present disclosure relates to a tomato plant, a tomato fruit, and a method of cultivating a tomato plant.

2. Description of the Related Art

In recent years, an artificial light type plant factory in which a plant such as a vegetable is cultivated by using an artificial light source such as an LED (Light Emitting Diode) in a closed space has attracted attention.
Cultivation of plants in an artificial light type plant factory is not affected by the climate and the weather, and the problem of labor shortage can be solved. Therefore, plants can be cultivated throughout a year under certain conditions.
For example, as the artificial light type plant factory, JP5330162B suggests a closed type plant factory suitable for cultivation of a tall (long-stem) plant such as tomato.
A tomato plant has a main branch, and after main leaves are differentiated in about eight stages, flower buds are differentiated and a first stage flower cluster (also referred to as first flower cluster) is settled, and JP2016-202072 suggests a light emitting device that reduces a number of stages of main leaves of a tomato plant, sets a settlement position of a first flower cluster at a low position, and can be used in an artificial light type plant factory and the like.

**SUMMARY OF THE INVENTION**

The present inventors found that a method of cultivating a tomato plant obtained by using a closed type plant factory disclosed in JP5330162B and a light emitting device disclosed in JP2016-202072 has room for improvement from a viewpoint of number of harvests per unit volume (space utilization efficiency) and quality of harvested tomatoes. Then, the present inventors newly found that by making a specific relationship between a sum (X) of the number of flower clusters and fruit clusters contained in one of a main branch or a side branch of the tomato plant and a length (L) in cm units from a planting surface to a growth point of the main branch or the side branch, it is possible to improve the space utilization efficiency and the quality of the harvested tomato fruit in cultivation of the tomato plant.
The present disclosure has been made on the above finding, and an object that an embodiment according to the present disclosure is achieved is to provide a tomato plant capable of being cultivated at a high space utilization efficiency and capable of harvesting a high-quality tomato fruit.
In addition, an object that an embodiment according to the present disclosure is to provide a tomato fruit harvested from the tomato plant and a method of cultivating the tomato plant.

<1> A tomato plant that satisfies Formula (1).

$$6.0 \le L/X \le 30.0 \ ... \ (1)$$

In the formula, in a case where the tomato plant does not have a side branch, L represents a length in cm unit from a planting surface to a growth point of a main branch, and X represents a sum of the number of flower clusters and fruit clusters of the main branch.
In a case where the tomato plant has a side branch, L represents a length in cm unit from the planting surface to the growth point of one main branch or side branch, and X represents a sum of the number of flower clusters and fruit clusters of the one main branch or side branch.
Here, X is an integer of 2 or more.

<2> The tomato plant according to <1>, in which in Formula (1), L is 100 or less.
<3> The tomato plant according to <1> or <2>, in which in Formula (1), X is an integer of 10 or less.
<4> The tomato plant according to any one of <1> to <3>, which satisfies Formula (2).

$$11.0 \le L/X \le 22.0 \ ... \ (2)$$

In the formula, in a case where the tomato plant does not have a side branch, L represents a length in cm unit from a planting surface to a growth point of a main branch, and X represents a sum of the number of flower clusters and fruit clusters of the main branch.

In a case where the tomato plant has a side branch, L represents a length in cm unit from the planting surface to the growth point of one main branch or side branch, and X represents a sum of the number of flower clusters and fruit clusters of the one main branch or side branch.

Here, X is an integer of 2 or more.

<5> A tomato fruit which is harvested from the tomato plant according to any one of <1> to <4> and contains 10 mg or more of lycopene per 100 g by weight.

<6> The tomato fruit according to <5>, in which a Brix sugar content is 7% by mass or more.

<7> A method of cultivating a tomato plant, at least including: a step of irradiating a ground part of a first tomato plant with artificial light having a light intensity of 200 $\mu$mol/m$^2$·s to 800 $\mu$mol/m$^2$·s, in which a second tomato plant that satisfies Formula (1) is cultivated from the first tomato plant.

$$6.0 \leq L/X \leq 30.0 \ldots (1)$$

In the formula, in a case where the second tomato plant does not have a side branch, L represents a length in cm unit from a planting surface to a growth point of a main branch, and X represents a sum of flower clusters and fruit clusters of the main branch.

In a case where the second tomato plant has a side branch, L represents a length in cm unit from the planting surface to the growth point of one main branch or side branch, and X represents a sum of the number of flower clusters and fruit clusters of the one main branch or side branch.

Here, X is an integer of 2 or more.

<8> The method of cultivating a tomato plant according to <7>, in which irradiation of the ground part of the first tomato plant with the artificial light is performed from a side surface direction of the tomato plant.

<9> The method of cultivating a tomato plant according to <7> or <8>, in which a light-dark cycle of the artificial light with which the ground part of the first tomato plant is irradiated is controlled, and a ratio of a light period time to a dark period time is 1 to 3.

<10> The method of cultivating a tomato plant according to any one of <7> to <9>, in which a cultivation temperature of the first tomato plant in a light period is 23°C to 33°C, and the cultivation temperature of the first tomato plant in a dark period is 15°C to 22°C.

<11> The method of cultivating a tomato plant according to any one of <7> to <10>, in which a carbon dioxide concentration of the first tomato plant in a cultivation environment is 300 ppm or more.

<12> The method of cultivating a tomato plant according to any one of <7> to <11>, in which a cultivation device including one or more selected from a light source for irradiating the ground part of the first tomato plant with the artificial light from at least one of an upper surface direction or a side surface direction, a hydroponic cultivation mechanism, and a temperature/humidity control mechanism is used.

According to one embodiment of the present disclosure, it is possible to provide a tomato plant capable of being cultivated with high space utilization efficiency and capable of harvesting a high-quality tomato fruit.

In addition, according to one embodiment of the present disclosure, it is possible to provide a tomato fruit harvested from the tomato plant and a method of cultivating the tomato plant.

## BRIEF DESCRIPTION OF THE DRAWINGS

Fig. 1 is a schematic diagram showing an example of a tomato plant according to the present disclosure.

Fig. 2 is a schematic diagram for explaining a method of measuring a light intensity.

Fig. 3 is a schematic diagram for explaining a method of measuring a light intensity.

## DESCRIPTION OF THE PREFERRED EMBODIMENTS

Hereinafter, embodiments for carrying out the present disclosure will be described in detail. However, the present invention is not limited to the following embodiments. In the following embodiments, the components are not essential unless otherwise specified. The same applies to numerical values and the ranges, and does not limit the present disclosure.

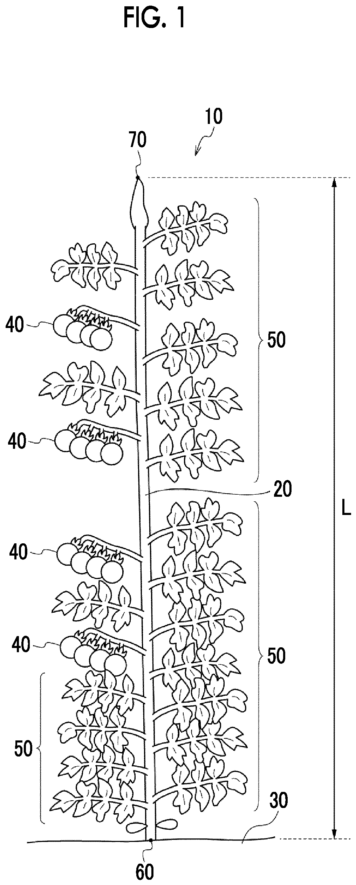

In the present disclosure, the "tomato plant" and the "second tomato plant" are tomato plants that satisfy Formula (1), and as shown in Fig. 1 (tomato plant in Fig. 1 is represented by reference numeral 10), has at least two main branches 20 and has two or more flower clusters and fruit clusters. In addition, in Fig. 1, reference numeral 40 indicates a flower cluster or fruit cluster, and reference numeral 50 indicates a main leaf. In addition, in Fig. 1, reference numeral 60 indicates a contact point between the tomato plant and the planting surface.

In the present disclosure, the "first tomato plant" means a plant after germination of tomato seeds and before becoming the tomato plant or the second tomato plant according to the present disclosure. The first tomato plant includes a first tomato plant before planting and a first tomato plant that does not satisfy Formula (1) even after planting. In addition, the planting means that a temporarily planted first tomato plant is transferred to a final place where main cultivation is performed.

In the present disclosure, the "side branch" means a branch in which a lateral bud generated from a leaf of a main branch or a base of a stem is extended, and a state in which a side branch is not present is referred to as one-tailoring, a state in which one or more side branches are included is referred to as a plurality of tailoring, a state in which one side branch is included is referred to as two-tailoring, and a state in which two side branches are included is referred to as three-tailoring.

In the present disclosure, as shown in Figs. 1 to 3, the "planting surface" means a surface of a support in which a tomato plant 10, a first tomato plant 90, or a second tomato plant (not shown), and is represented by reference numeral 30.

In the present disclosure, as shown in Fig. 1, the "growth point" means a tip of a main branch 20 or a side branch (not shown) of the tomato plant 10, and is represented by reference numeral 70 in Fig. 1.

In the present disclosure, the "length in cm unit from a planting surface to a growth point of a main branch" means a length from a contact point 60 between the tomato plant 10 and the planting surface to a growth point 70 of the main branch, measured along a shape of the main branch. The same applies to a case of a side branch.

In the present disclosure, the "ground part of the first tomato plant" refers to a portion above the planting surface.

In the present disclosure, the "Brix sugar content" refers to a value in which a refractive index measured at 20°C using a sugar content meter or a refractometer is converted into % by mass of a sucrose solution based on a conversion table of the International Commission for Uniform Methods of Sugar Analysis (ICUMSA).

In the present disclosure, the term "step" includes not only an independent step but also a step as long as a desired purpose of the step is achieved even in a case where the step cannot be clearly distinguished from other steps.

In the present disclosure, "% by mass" and "% by weight" are synonymous, and "parts by mass" and "parts by weight" are synonymous.

(Tomato plant)

A tomato plant according to the present disclosure satisfies Formula (1). In addition, in L/X, the number of the second digit after a decimal point is rounded off.

$$6.0 \leq L/X \leq 30.0 \ ... \ (1)$$

In the formula, in a case where the tomato plant does not have a side branch, L represents a length in cm unit from a planting surface to a growth point of a main branch, and X represents a sum of the number of flower clusters and fruit clusters of the main branch.

In a case where the tomato plant has a side branch, L represents a length in cm unit from the planting surface to the growth point of one main branch or side branch, and X represents a sum of the number of flower clusters and fruit clusters of the one main branch or side branch.

Here, X is an integer of 2 or more.

According to the tomato plant according to the present disclosure, it is possible to cultivate the tomato plant with high space utilization efficiency and to harvest a high-quality tomato fruit. The reason why the above effect is exhibited is presumed as follows, but is not limited thereto.

In the tomato plant according to the present disclosure, since a distance between flower clusters, between fruit clusters, or between a flower cluster and a fruit cluster, which are adjacent to each other, is short, and the number of harvests (number of harvests per unit volume) with respect to the length of the tomato plant is high, the space utilization efficiency is high.

In addition, since nutrients that should have been used for the growth of the tomato plant in the length direction can be used for the tomato fruit, the tomato fruit harvested from the tomato plant has high quality.

In the tomato plant according to the present disclosure, as the L/X is 6.0 or more, fruiting is favorably generated in the flower cluster. In addition, as the L/X is 30.0 or less, it is possible to improve the space utilization efficiency in the cultivation of the tomato plant, and the first tomato plant and the second tomato plant, which will be described later, and it is possible to improve the quality of the harvested tomato fruit.

An L/X adjustment method can be performed, for example, by adjusting a light intensity of a light source used for cultivating the first tomato plant.

In Formula (1), L is preferably 100 or less, more preferably 95 or less, and still more preferably 90 or less. By setting L to 100 or less, it is possible to further improve the space utilization efficiency in the cultivation of the tomato plant according to the present disclosure.

In addition, L is preferably 20 or more. By setting L to 20 or more, it is possible to improve the number of harvests of the tomato fruits.

In Formula (1), the fact that X is an integer of 2 or more means that the tomato plant has grown to a certain extent or more.

X is preferably an integer of 10 or less, more preferably an integer of 7 or less, and still more preferably an integer of 5 or less. By setting X to an integer of 10 or less, it is possible to further improve the quality of the harvested tomato fruit.

In addition, X is preferably an integer of 3 or more. By setting X to an integer of 3 or more, it is possible to improve the number of harvests of the tomato fruits.

It is preferable to pinch the tomato plant, the first tomato plant, or the second tomato plant in order to set X in the above range.

In the present disclosure, "pinching" means picking a bud at a growth point of a tomato plant, a first tomato plant, or a second tomato plant, and stopping the growth of a stem of the tomato plant.

A support that supports the tomato plant, the first tomato plant, and the second tomato plant is not particularly limited, and in a case where the tomato plant, the first tomato plant, and the second tomato plant are cultivated by hydroponic cultivation, for example, a support base provided with urethane foam, rockwool, water-retaining sheet, and the like can be used. In addition, in a case where the tomato plant, the first tomato plant, and the second tomato plant are cultivated by soil cultivation, for example, culture soil and the like can be used. In addition, even in the case of performing cultivation by soil cultivation, the urethane foam and the like may be used.

In addition, a support member such as a post or a string for attracting hanging for the tomato plant, the first tomato plant, or the second tomato plant may be used.

From a viewpoint of space utilization efficiency and quality of harvested tomato fruits, the tomato plant according to the present disclosure preferably satisfies Formula (2), and more preferably satisfies Formula (3).

$$11.0 \leq L/X \leq 22.0 \; ... \; (2)$$

$$11.0 \leq L/X \leq 15.0 \; ... \; (3)$$

In Formulas (2) and (3), L and X are as described above.

The tomato plant according to the present disclosure has at least a main branch, but may have one or more side branches.

In a case where the tomato plant does not have a side branch, L represents a length in cm unit from a planting surface to a growth point of the main branch, and X represents a sum of the number of flower clusters and fruit clusters of the main branch.

In a case where the tomato plant has one or more side branches, L represents the length in cm unit from the planting surface to the growth point of one main branch or one side branch, and X represents the sum of flower clusters and fruit clusters of the one main branch or side branch. That is, in a case where the tomato plant has one or more side branches, the one main branch or side branch may satisfy the Formula (1), and the other branches may not satisfy Formula (1). From the viewpoint of space utilization efficiency in the cultivation of the tomato plant and quality of the harvested tomato fruit, all the main branches and the side branches of the tomato plant preferably satisfy Formula (1).

From the viewpoint of the quality of the harvested tomato fruit, the number of the side branches is preferably 3 or less, more preferably 1 or less, and the tomato plant further more preferably has no side branch.

The number of fruiting in each flower cluster of the main branch or the side branch is preferably 1 to 7, and more preferably 2 to 5. By setting the number of fruiting within the numerical value range, it is possible to further improve the quality of the harvested tomato fruit. Specifically, in the tomato plant according to the present disclosure in which a distance between flower clusters (or fruit clusters), which are adjacent to each other, is short, it is possible to effectively prevent the flower clusters (fruit clusters) from coming into contact with each other and causing crushing or the like, and it is possible to improve the quality of the harvested tomato fruit.

In addition, in a case where the number of fruiting is large, it is preferable to pick the fruit.

In the tomato plant, the first tomato plant, and the second tomato plant according to the present disclosure, it is preferable that main leaves below the flower clusters where flowering is confirmed are defoliated. Specifically, as for the main leaves below the flower clusters where flowering is confirmed, 3 or more and 5 or less leaves are defoliated, and more preferably 2 or more and 4 or less leaves are defoliated. By maintaining the tomato plant in the state by defoliation, it is possible to improve the quality of the harvested tomato fruit. In addition, by performing the defoliation, it is possible to avoid mutual shading, and to improve the cultivation efficiency of the tomato plant, the first tomato plant, and the second tomato plant.

(Tomato fruit)

The tomato fruit according to the present disclosure is harvested from the tomato plant and contains 10 mg or more of lycopene per 100 g by weight. The lycopene content is preferably 15 mg/100 g or more, and more preferably 17 mg/100 g or more.

The Brix sugar content of the tomato fruit according to the present disclosure is preferably 3.5% by mass or more, more preferably 4% by mass or more, and still more preferably 7% by mass or more.

In the present disclosure, the Brix sugar content and the lycopene amount of the tomato fruit are obtained as an average value of values measured in four portions on an equator in which a diameter of the tomato fruit is the largest, which are four portions on which a line connecting two portions is orthogonal to a line connecting other two portions, using a fruit vegetable quality evaluation device Fruit Selector (model: K-BA800, manufactured by Kubota Co., Ltd.).

Specifically, the lycopene amount obtained by using Fruit Selector is measured as follows.

First, a calibration curve is created by correlating the lycopene amount of the tomato fruit measured by high-speed liquid chromatography (HPLC) with the lycopene amount measured by Fruit Selector. Next, the lycopene amount is measured by converting the lycopene amount measured by using Fruit Selector into the lycopene amount measured by HPLC based on the calibration curve.

The variety of the tomato fruit varieties according to the present disclosure is not particularly limited, and can be a known variety of the tomato fruit in the related art. In addition, the type such as large tomato, midi tomato, mini tomato, and the like is not particularly limited.

(Method of cultivating tomato plant)

A method of cultivating a tomato plant according to the present disclosure includes at least a step of irradiating a first tomato plant with an artificial light having a light intensity of 200 $\mu$mol/m$^2$·s to 800 $\mu$mol/m$^2$·s, and cultivates a second tomato plant that satisfies Formula (1) from the first tomato plant. The second tomato plant preferably satisfies Formula (2), and more preferably satisfies Formula (3).

According to the method of cultivating a tomato plant according to the present disclosure, it is possible to cultivate a tomato plant with high space utilization efficiency and to harvest a high-quality tomato fruit. The reason why the above effect is exhibited is presumed as follows, but is not limited thereto.

The method of cultivating a tomato plant according to the present disclosure includes at least a step of irradiating the first tomato plant with an artificial light having a light intensity of 200 $\mu$mol/m$^2$·s to 800 $\mu$mol/m$^2$·s, cultivates a second tomato plant that satisfies Formula (1) from the first tomato plant, and as the first tomato plant is irradiated with an artificial light having an intensity within the above numerical value range, a distance between flower clusters, between fruit clusters, or between a flower cluster and a fruit cluster, which are adjacent to each other, of a main branch or a side branch, is short, and the number of harvests with respect to a length of the main branch or the side branch of the second tomato plant is improved, and thus the space utilization efficiency becomes high.

In addition, since nutrients that should have been used for the growth of the main branch or the side branch of the first tomato plant and the second tomato plant in the length direction can be used for the tomato fruit, the tomato fruit harvested from the second tomato plant has high quality.

In the method of cultivating a tomato plant according to the present disclosure, by setting the light intensity of the artificial light with which the first tomato plant is irradiated to 200 $\mu$mol/m$^2$·s or more, a distance between flower clusters, between fruit clusters, or between a flower cluster and a fruit cluster, which are adjacent to each other, of the obtained second tomato plant, is short, and the number of harvests with respect to the length of the second tomato plant is improved, and thus it is possible to heighten the space utilization efficiency and to improve the quality of the harvested tomato fruit.

In addition, by setting the light intensity of the artificial light with which the first tomato plant is irradiated to 800 $\mu$mol/m$^2$·s or less, fruiting is favorably generated in the flower cluster.

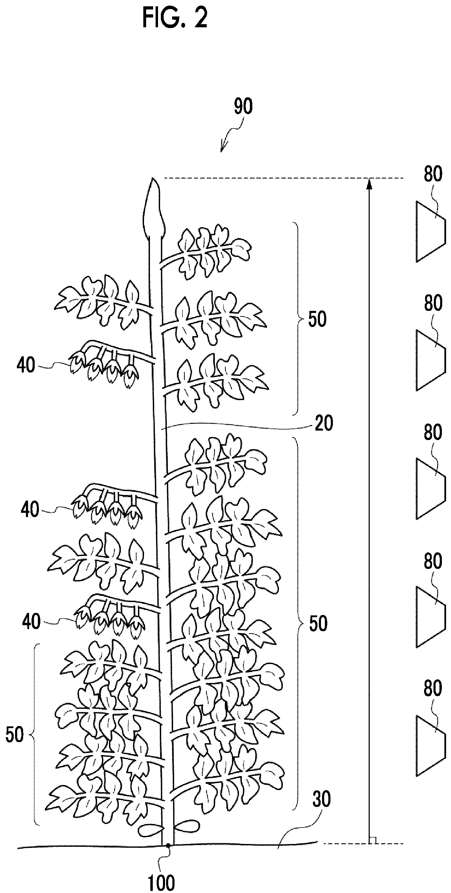

In the present disclosure, as shown in Fig. 2, the light intensity is calculated by measuring a light intensity by disposing a measuring device toward light sources 80 every 10 cm from a contact point 100 between a first tomato plant 90 and a planting surface 30 to a height of a light source installed at an uppermost stage in a direction perpendicular to the planting surface 30, and obtaining an average value of the values.

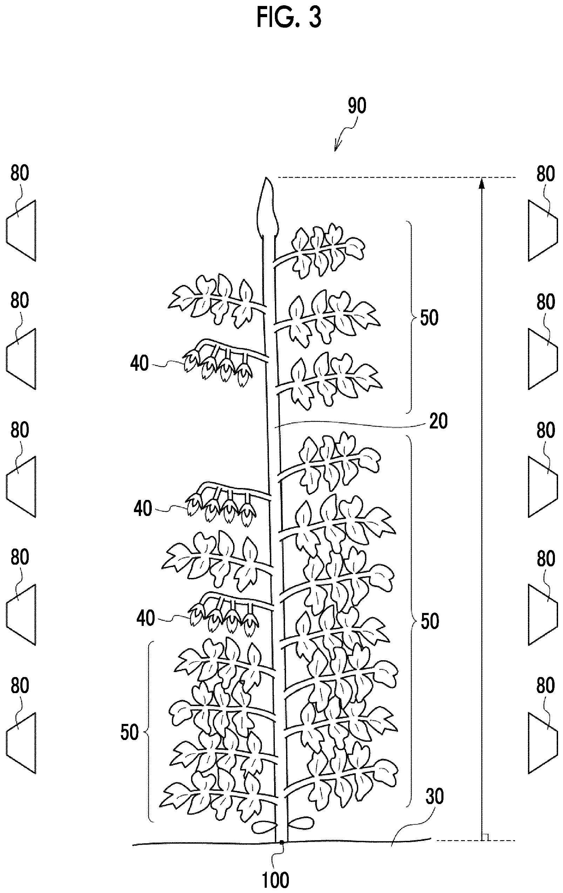

As shown in Fig. 3, in a case where the light sources 80 are disposed on both side surfaces of the first tomato plant, the light intensity is calculated by measuring a sum of the light intensity at each position by disposing the measuring device toward each of the light sources 80 on both side surfaces every 10 cm from the contact point 100 between the first tomato plant 90 and the planting surface 30 to the height of the light source at an uppermost stage in a direction perpendicular to the planting surface 30, and obtaining an average value of the values.

As the measuring device, for example, a photon sensor (LI-109, manufactured by LI-COR) and the like can be used.

The light intensity is preferably measured before the start of cultivation of the first tomato plant in order to avoid the influence of the shadow of the first tomato plant. In a case where the light intensity is measured before the start of cultivation of the first tomato plant, a position of the contact point between the first tomato plant and the planting surface is determined based on a planned disposition position of the first tomato plant.

In the present disclosure, the light intensity means a light intensity in the light period.

In sunlight, the light intensity increases up to about 2,000 $\mu$mol/m$^2$·s in one day, but a time for achieving the light intensity is extremely short (about 1 hour to 2 hours). In addition, the irradiation of the first tomato plant with sunlight is from an upper surface direction, and the light intensity of sunlight with which a part of the first tomato plant is irradiated due to the shadow of the main leaves and the like of the first tomato plant becomes a low value. For the above-mentioned reasons, the light intensity in a case where the first tomato plant is cultivated using sunlight is not an appropriate value, a distance between flower clusters, between fruit clusters, or between a flower cluster and a fruit cluster, which are adjacent to each other, is short, and a long-stem tomato plant that does not satisfy Formula (1) is obtained.

From a viewpoint of space utilization efficiency, quality of harvested tomato fruits, and number of harvests, the light intensity is preferably 220 $\mu$mol/m$^2$·s to 780 $\mu$mol/m$^2$·s, more preferably 300 $\mu$mol/m$^2$·s to 750 $\mu$mol/m$^2$·s, and further more preferably 400 $\mu$mol/m$^2$·s to 720 $\mu$mol/m$^2$·s. It should be noted that the light intensity is for the light period.

The first tomato plant may be irradiated with the artificial light from the upper surface direction or the side surface direction, but the distance between adjacent flower clusters and the like can be shorter, and the space utilization efficiency can be further improved, and thus it is preferable to perform irradiation with the artificial light from the side surface direction. In addition, by performing irradiation with the artificial light from the side surface direction of the first tomato plant, the light can efficiently reach the leaves, which are the photosynthetic organs, and thus it is possible to further decrease the light for irradiation, and to improve the cultivation efficiency of the obtained second tomato plant.

In addition, the irradiation with the artificial light may be performed from both the side surface direction and the upper surface direction.

It is preferable that a cycle of the light period and the dark period (hereinafter, referred to as a light-dark cycle) of the artificial light for irradiation is controlled.

From a viewpoint of the quality of the harvested tomato fruit, a ratio of the light period time to the dark period time (light period time/dark period time) is preferably 1 to 3, and more preferably 1.2 to 2.2.

In the present disclosure, the "light period" means a period during which the first tomato plant is irradiated with the light source. In addition, in the present disclosure, the "dark period" means a period during which the first tomato plant is not irradiated with the light source.

The light source of the artificial light for irradiation is not particularly limited, and examples of the light source include an LED and a fluorescent lamp. However, in the method of cultivating a tomato plant according to the present disclosure, it is preferable to use an LED.

One type of LED may be used, or two or more types of LEDs may be used.

The LED may emit visible light such as red, blue, and yellow, or may emit invisible light of ultraviolet light (wavelength of 380 nm or less) or infrared light (wavelength of 780 nm or more). However, from a viewpoint of promoting photosynthesis of the first tomato plant, the LED preferably emits light in a wavelength range of 400 nm to 700 nm.

Since chlorophyll, which is a photosynthetic pigment, has light absorption peaks in a wavelength range of approximately 400 nm to 500 nm and a wavelength range of 600 nm to 700 nm, from a viewpoint of promoting photosynthesis, it is preferable to use an LED including an element that emits light having a peak wavelength in a wavelength range of 400 nm to 500 nm and an LED including an element that emits light having a peak wavelength in a wavelength range of 600 nm to 700 nm in combination.

In a case where the LED including an element that emits light having a peak wavelength in a wavelength range of 400 nm to 500 nm and the LED including an element that emits light having a peak wavelength in a wavelength range of 600 nm to 700 nm are used in combination, light composition is adjusted such that a ratio of a light intensity of light having a peak wavelength in a wavelength range of 600 nm to 700 nm to a light intensity of light having a peak wavelength in a wavelength range of 400 nm to 500 nm (light intensity of light having peak wavelength in wavelength range of 600 nm to 700 nm/light intensity of light having peak wavelength in wavelength range of 400 nm to 500 nm) is preferably 1.5 to 4, and more preferably 1.8 to 3.

In the present disclosure, the peak wavelength means a wavelength at which the emission intensity is maximized in one peak of the spectral waveform.

In addition, for example, an LED that emits pseudo-white visible light can be used. The LED that emits pseudo-white visible light is a combination of an LED that emits blue visible light and a yellow phosphor that is excited by the visible light.

In a case where the LED that emits pseudo-white visible light is used, the obtained spectral waveform has a first peak wavelength in a wavelength of 400 nm to 480 nm and a second peak wavelength in a wavelength of 570 nm to 600 nm. In addition, the peak including the second peak wavelength exists over a wide wavelength range (425 nm to 760 nm).

In a case where the LED that emits pseudo-white visible light is used, a light composition in which a correlated color temperature of the light is 3000 K to 6000 K is preferable, and a light composition in which a correlated color temperature of the light is 4000 K to 5500 K is more preferable.

Examples of the LED that emits pseudo-white visible light include a plant growth LED white type (PGL-NE-200NWD), manufactured by Ryoden Trading Company, Limited.

In the present disclosure, the "correlated color temperature" is a value obtained in accordance with Japanese Industrial Standards (JIS) Z 8725: issued in 2015 (measurement method for distribution temperature of light source and color temperature/correlated color temperature).

In addition, for example, an LED that emits red visible light, an LED that emits blue visible light, an LED that emits invisible light, and an LED that emits pseudo-white visible light can be used in combination.

In a case where the LED that emits red visible light, the LED that emits blue visible light, the LED that emits invisible light, and the LED that emits pseudo-white visible light are used in combination, the obtained spectral waveform has a first peak wavelength in a wavelength of 400 nm to 480 nm, has a second peak wavelength in a wavelength of 650 nm to 700 nm, and has a third peak wavelength in a wavelength of 730 nm to 750 nm. In addition, the spectral waveform has an emission intensity value of a certain value or higher in a wavelength range of 425 nm to 760 nm.

The cultivation temperature of the first tomato plant is not particularly limited, but is preferably 23°C to 33°C, and more preferably 25°C to 32°C in the light period. By setting the cultivation temperature in the light period within the numerical value range, it is possible to improve the quality of the harvested tomato fruit.

In addition, the cultivation temperature of the first tomato plant in the dark period is preferably 15°C to 22°C, and more preferably 16°C to 20°C. By setting the cultivation temperature in the dark period within the numerical value range, it is possible to further improve the quality of the harvested tomato fruit.

In the present disclosure, the cultivation temperature is measured by disposing a thermometer at a position 1 cm away from the first tomato plant at an intermediate point between a contact point between the first tomato plant and the planting surface and a growth point. As the thermometer, for example, TR-74Ui manufactured by T & D Co., Ltd. and the attached sensors can be used.

A relative humidity in the cultivation environment of the first tomato plant is preferably 40% to 70%, and more preferably 50% to 65%. By setting the relative humidity in the cultivation environment of the first tomato plant within the numerical value range, it is possible to further improve the quality of the harvested tomato fruit.

In the present disclosure, the relative humidity is measured by disposing a humidity meter at a position 1 cm away from the first tomato plant at an intermediate point between the contact point between the first tomato plant and the planting surface and the growth point. As the humidity meter, for example, TR-74Ui manufactured by T & D Co., Ltd. and the attached sensors can be used.

A carbon dioxide concentration in the cultivation environment of the first tomato plant is preferably 300 ppm or more, more preferably 400 ppm or more, further more preferably 800 ppm or more, and particularly preferably 1,000 ppm or more. By setting the carbon dioxide concentration in the cultivation environment of the first tomato plant within the numerical value range, it is possible to further improve the quality of the harvested tomato fruit.

In the present disclosure, the carbon dioxide concentration is measured by disposing a carbon dioxide concentration meter at a position 1 cm away from the first tomato plant at an intermediate point between the contact point between the first tomato plant and the planting surface and the growth point. As the carbon dioxide concentration meter, for example, LI-850 manufactured by LI-COR, Inc. can be used.

The first tomato plant may be cultivated by soil cultivation or hydroponic cultivation, but from a viewpoint of the number of harvests of the tomato fruits, the hygiene of the cultivation environment, and the like, hydroponic cultivation is preferably performed.

In a case where the first tomato plant is cultivated by soil cultivation, the soil such as culture soil corresponds to a support that supports a root part of the first tomato plant.

In a case where the first tomato plant is cultivated by hydroponic cultivation, for example, a support base provided with urethane foam, rockwool, and water-retaining sheet, and the like can be used as the support that supports a root part.

In a case where the first tomato plant is cultivated by hydroponic cultivation, a method of supplying a liquid fertilizer is not particularly limited, and examples of the method include a flooded hydroponic cultivation in which a support is dipped in the liquid fertilizer for cultivation, a spray type hydroponic cultivation in which a liquid fertilizer is sprayed onto a support, and a drop type hydroponic cultivation in which a liquid fertilizer is added dropwise onto a root part or a support, and the like.

The liquid fertilizer to be used is not particularly limited as long as the liquid fertilizer is suitable for tomato cultivation, and for example, a commercially available mixed liquid fertilizer (OAT House #1 manufactured by OAT Agrio CO., Ltd. and Home Hyponica manufactured by Kyowa Co., Ltd.) may be used by being dissolved, and diluted at a desired concentration, or may be used in combination of a single fertilizer based on a known fertilizer composition such as a garden formula and a Yamazaki formula.

A concentration of the liquid fertilizer can be obtained by using an EC (Electrical Conductivity) value as an index, and, from a viewpoint of the quality of the harvested tomato fruit, is preferably 1.0 ds/m to 8.0 ds/m, and more preferably 1.0 ds/m to 8.0 ds/m. In addition, the concentration of the liquid fertilizer may be changed in accordance with the growth of the first tomato plant.

An EC value is measured at 25°C by using an electric conductivity meter (for example, HI98131 manufactured by Hanna Instruments).

The method of cultivating a tomato plant according to the present disclosure is preferably performed using a cultivation device including one or more selected from a light source for irradiating a ground part of the first tomato plant with an artificial light from at least one of an upper surface direction or a side surface direction, a hydroponic cultivation mechanism, and a temperature/humidity control mechanism is used.

In addition, the cultivation device more preferably includes a liquid fertilizer supply mechanism, and further more preferably includes an EC value adjustment mechanism of the liquid fertilizer. In addition, the cultivation device further more preferably includes a mechanism that adjusts a light intensity of a light source, a light-dark cycle, a carbon dioxide concentration, and the like.

Examples

Hereinafter, embodiments will be specifically described with reference to Examples, but the embodiments are not limited to these Examples.

<Example 1>

Tomato seeds (variety: Momotaro York (registered trademark)) were sown on a 5 cm square rockwool (Yasaihana Pot, manufactured by Nippon Rockwool Corporation) sufficiently containing pure water, which is a support, and stored in the darkness under the conditions of a temperature of 28°C and a relative humidity of 70% for three days.

After the germination of the tomato seeds was confirmed, seedlings were raised for 27 days by a bottom liquid feeding method to obtain a first tomato plant. As the liquid fertilizer, Home Hyponica manufactured by Kyowa Co., Ltd. diluted 500-fold with pure water was used, and the liquid feeding was performed twice a day.

A white LED (PGL-NE-200NWD) manufactured by Ryoden Trading Company, Limited was used as a light source during the seedling raising period, a light intensity was set to 250 $\mu$mol/m$^2 \cdot$s, and irradiation was performed from the upper surface direction.

The obtained first tomato plant was cultivated using five light sources (number of one side) disposed on both side surfaces of the first tomato plant at an interval of 20 cm in a vertical direction, and a cultivation device including a flooded hydroponic cultivation mechanism and a temperature/humidity control mechanism under the following cultivation conditions to obtain a tomato plant (second tomato plant).

The light intensity was measured before cultivation of the first tomato plant.

Specifically, the light intensity was calculated by measuring a sum of the light intensity at each position by determining a position of a contact point between the first tomato plant and a planting surface based on a scheduled disposition position of the first tomato plant, disposing a photon sensor toward each of the light sources on both side surfaces every 10 cm from the contact point to the height of the light source at an uppermost stage in a direction perpendicular to the planting surface, and obtaining an average value of the values.

During the cultivation period, according to a standard method, one-tailoring was performed to arrange (lateral bud nipping, defoliation, and the like) and attract branches, and after five flower clusters (first to fifth flower clusters) settled on the main branch, and then it was confirmed that two main leaves were developed on an upper side of the fifth flower cluster, branches were pinched while leaving the main leaves.

Each fruit cluster was thinned such that the number of fruiting is 3, the tomato fruits born up to the fifth flower cluster were harvested, and cultivation was completed. A total number of harvests of the tomato fruits during the cultivation period was 15.

A length (L) from the planting surface to the growth point of the main branch was 90 cm, and the L/X was 18.0.

(Cultivation conditions)

·Light source: LED, CIVILIGHT, manufactured by Showa Denko Corporation (each light intensity is adjustable with fluorescent lamp type LED provided with an element that emits red light having a peak wavelength at a wavelength of 660 nm and an element that emits blue light having a peak wavelength at a wavelength of 450 nm, and a dimmer (UL9005 #01-0R) manufactured by Union Electronics Industrial Co., Ltd.)
·Light intensity: 250 $\mu$mol/m$^2$·s
·Light composition: The ratio of the light intensity of red light having a peak wavelength at a wavelength of 660 nm to the light intensity of blue light having a peak wavelength at a wavelength of 450 nm = 2[167([$\mu$mol/m$^2$·s)/83 ($\mu$mol/m$^2$·s)]: Described as light composition 1 in Table 1

·Light-dark cycle (light period/dark period): 14 hours/10 hours
·Temperature: 25°C (light period), 17°C (dark period)
·Relative humidity: 60%
·Carbon dioxide concentration: 400 ppm
·Fertilization method: Flooded hydroponic cultivation
·Liquid fertilizer: "Hyponica liquid fertilizer" manufactured by Kyowa Co., Ltd. is used by being diluted with pure water
·EC value of liquid fertilizer: 2.0 ds/m

<Example 2>

Tomato fruits were harvested in the same manner as Example 1, except that, after confirming that four flower clusters settled on the main branch and two main leaves developed on the upper side of the fourth flower cluster, the branches were pinched while leaving the main leaves, pinching was performed such that the number of fruiting on each flower cluster was four, and the cultivation conditions were changed as follows. A length (L) from the planting surface to the growth point of the main branch was 45 cm, and the L/X was 11.3. The number of harvests of the tomato fruits was 16.

(Cultivation conditions)

·Light source: LED, CIVILIGHT, manufactured by Showa Denko Corporation
·Light intensity: 500 $\mu$mol/m$^2$·s
·Light composition: The ratio of the light intensity of red light having a peak wavelength at a wavelength of 660 nm to the light intensity of blue light having a peak wavelength at a wavelength of 450 nm = 2[334 ($\mu$mol/m$^2$·s)/166 ($\mu$mol/m$^2$·s)]: Described as light composition 2 in Table 1
·Light-dark cycle (light period/dark period): 16 hours/8 hours
·Temperature: 25°C (light period), 19°C (dark period)
·Relative humidity: 60%
·Carbon dioxide concentration: 1,000 ppm
·Fertilization method: Drop type hydroponic cultivation
·Liquid fertilizer: "Hyponica liquid fertilizer" manufactured by Kyowa Co., Ltd. is used by being diluted with pure water
·EC value of liquid fertilizer: 3.5 ds/m

<Example 3>

Tomato fruits were harvested in the same manner as Example 1, except that, after confirming that three flower clusters settled on the main branch and two main leaves developed on the upper side of the third flower cluster, the branches were pinched while leaving the main leaves, pinching was performed such that the number of fruiting on each flower cluster was one, and the cultivation conditions were changed as follows. The length (L) from the planting surface to the growth point of the main branch was 19 cm, and the L/X was 6.3. The number of harvests of the tomato fruits was three.

(Cultivation conditions)

·Light source: LED, CIVILIGHT manufactured by Showa Denko Corporation
·Light intensity: 800 $\mu$mol/m$^2$·s
·Light composition: The ratio of the light intensity of red light having a peak wavelength at a wavelength of 660 nm to the light intensity of blue light having a peak wavelength at a wavelength of 450 nm = 2[533 ($\mu$mol/m$^2$·s)/267 ($\mu$mol/m$^2$·s)]: Described as light composition 3 in Table 1.
·Light-dark cycle (light period/dark period): 14 hours/10 hours
·Temperature: 25°C (light period), 17°C (dark period)
·Relative humidity: 60%
·Carbon dioxide concentration: 400 ppm
·Fertilization method: Flooded hydroponic cultivation
·Liquid fertilizer: "Hyponica liquid fertilizer" manufactured by Kyowa Co., Ltd. is used by being diluted with pure water
·EC value of liquid fertilizer: 1.5 ds/m

<Example 4>

Tomato fruits were harvested in the same manner as Example 1, except that, after confirming that three flower clusters settled on the main branch and two main leaves developed on the upper side of the third flower cluster, the branches were pinched while leaving the main leaves, pinching was performed such that the number of fruiting on each flower cluster was three, and the cultivation conditions were changed as follows. The length (L) from the planting surface to the growth point of the main branch was 88 cm, and the L/X was 29.3. The number of harvests of the tomato fruits was nine.

(Cultivation conditions)

·Light source: Plant growth LED white type, PGL-NE-200NWD, manufactured by Ryoden Trading Company, Limited
·Light intensity: 200 $\mu$mol/m$^2$·s
·Light composition: Correlated color temperature = 4000K: Described in light composition 4 in Table 1
·Light-dark cycle (light period/dark period): 14 hours/10 hours
·Temperature: 25°C (light period), 17°C (dark period)
·Relative humidity: 60%
·Carbon dioxide concentration: 800 ppm
·Fertilization method: Flooded hydroponic cultivation
·Liquid fertilizer: "Hyponica liquid fertilizer" manufactured by Kyowa Co., Ltd. is used by being diluted with pure water
·EC value of liquid fertilizer: 1.5 ds/m

<Example 5>

Tomato fruits were harvested in the same manner as Example 1, except that, after confirming that four flower clusters settled on the main branch and two main leaves developed on the upper side of the fourth flower cluster, the branches were pinched while leaving the main leaves, pinching was performed such that the number of fruiting on each flower cluster was five, and the cultivation conditions were changed as follows. The length (L) from the planting surface to the growth point of the main branch was 95 cm, and the L/X was 23.8. The number of harvests of the tomato fruits was 20.

(Cultivation conditions)

·Light source: Plant growth LED white type, PGL-NE-200NWD, manufactured by Ryoden Trading Company, Limited
·Light intensity: 300 $\mu$mol/m$^2$·s
·Light composition: Correlated color temperature = 4000K: Described in light composition 4 in Table 1
·Light-dark cycle (light period/dark period): 16 hours/8 hours
·Temperature: 30°C (light period), 17°C (dark period)
·Relative humidity: 60%
·Carbon dioxide concentration: 1,000 ppm
·Fertilization method: Drop type hydroponic cultivation
·Liquid fertilizer: "Hyponica liquid fertilizer" manufactured by Kyowa Co., Ltd. is used by being diluted with pure water

·EC value of liquid fertilizer: 3.5 ds/m

<Example 6>

Tomato fruits were harvested in the same manner as Example 1, except that, after confirming that five flower clusters settled on the main branch and two main leaves developed on the upper side of the fifth flower cluster, the branches were pinched while leaving the main leaves, pinching was performed such that the number of fruiting on each flower cluster was four, and the cultivation conditions were changed as follows. The length (L) from the contact point between the planting surface of a ripe tomato plant and a surface of the support to the growth point of the main branch was 65 cm, and the L/X was 13.0. The number of harvests of the tomato fruits was 20.

(Cultivation conditions)

·Light source: Plant growth LED 4-color type, PGL-200DWBF26D, manufactured by Ryoden Trading Company, Limited
·Light intensity: 500 $\mu$mol/m$^2$·s
·Light composition: In accordance with light emission behavior of the LED: Described as light composition 5 in Table 1
·Light-dark cycle (light period/dark period): 16 hours/8 hours
·Temperature: 27°C (light period), 19°C (dark period)
·Relative humidity: 60%
·Carbon dioxide concentration: 1,000 ppm
·Fertilization method: Drop type hydroponic cultivation
·Liquid fertilizer: "Hyponica liquid fertilizer" manufactured by Kyowa Co., Ltd. is used by being diluted with pure water
·EC value of liquid fertilizer: Initially, a liquid fertilizer having an EC value of 1.5 ds/m was used, sodium chloride was added to the liquid fertilizer until flowering of the third flower cluster, and the EC value of the liquid fertilizer was gradually increased to 3.5 ds/m. After the flowering of the third flower cluster, a liquid fertilizer of 8.0 ds/m was used.

<Example 7>

Tomato fruits were harvested in the same manner as Example 1, except that, after confirming that three flower clusters settled on the main branch and two main leaves developed on the upper side of the third flower cluster, the branches were pinched while leaving the main leaves, pinching was performed such that the number of fruiting on each flower cluster was two, and the cultivation conditions were changed as follows. The length (L) from the contact point between the planting surface of a ripe tomato plant and a surface of the support to the growth point of the main branch was 65 cm, and the L/X was 21.7. The number of harvests of the tomato fruits was 6.

(Cultivation conditions)

·Light source: Plant growth LED white type, PGL-NE-200NWD, manufactured by Ryoden Trading Company, Limited
·Light intensity: 500 $\mu$mol/m2·s
·Light composition: Correlated color temperature = 4000K: Described in light composition 4 in Table 1
·Light-dark cycle (light period/dark period): 16 hours/8 hours
·Temperature: 25°C (light period), 19°C (dark period)
·Relative humidity: 60%
·Carbon dioxide concentration: 1,000 ppm
·Fertilization method: Drop type hydroponic cultivation
·Liquid fertilizer: "Hyponica liquid fertilizer" manufactured by Kyowa Co., Ltd. is used by being diluted with pure water
·EC value of liquid fertilizer: 6.0 ds/m

<Comparative Example 1>

Seedlings obtained in Example 1 were cultivated in a greenhouse (April to July, Kanagawa Prefecture) according to a standard method under the following cultivation conditions. In addition, after it was confirmed that three flower clusters settled on the main branch, and then two main leaves developed on the upper side of the third flower cluster, branches were pinched while leaving the main leaves.

Each fruit cluster was thinned such that the number of fruiting is 4, the tomato fruits born up to the third flower cluster were harvested, and cultivation was completed. The total number of harvests of the tomato fruits during the cultivation period was 12. The length (L) from the planting surface to the growth point of the main branch was 106 cm, and the L/X was 35.3.

(Cultivation conditions)

·Light source: Sun
·Temperature: 35°C (light period), 18°C (dark period)
·Relative humidity: 60%
·Carbon dioxide concentration: 400 ppm
·Fertilization method: Drop type hydroponic cultivation
·Liquid fertilizer: "Hyponica liquid fertilizer" manufactured by Kyowa Co., Ltd. is used by being diluted with pure water
·EC value of liquid fertilizer: 2.0 ds/m

<Comparative Example 2>

When seedlings were cultivated in the same manner as in Example 1, except that the cultivation conditions were changed as follows, fruiting was not generated, and tomato fruits could not be harvested. The number of settled flower clusters (X) was 3, the length (L) from the planting surface to the growth point of the main branch was 15 cm, and the L/X was 5.0.

(Cultivation conditions)

·Light source: LED, CIVILIGHT manufactured by Showa Denko Corporation
·Light intensity: 900 $\mu$mol/m$^2$·s
·Light composition: The ratio of the light intensity of red light having a peak wavelength at a wavelength of 660 nm to the light intensity of blue light having a peak wavelength at a wavelength of 450 nm = 2[600 ($\mu$mol/m$^2$·s)/300 ($\mu$mol/m$^2$·s)]: Described as light composition 6 in Table 1
·Light-dark cycle (light period/dark period): 14 hours/10 hours
·Light-dark cycle (light period/dark period): 14 hours/10 hours
·Temperature: 25°C (light period), 17°C (dark period)
·Relative humidity: 60%
·Carbon dioxide concentration: 400 ppm
·Fertilization method: Flooded hydroponic cultivation
·Liquid fertilizer: "Hyponica liquid fertilizer" manufactured by Kyowa Co., Ltd. is used by being diluted with pure water
·EC value of liquid fertilizer: 2.0 ds/m

<<Measurement of Brix sugar content and lycopene amount>>

The Brix sugar content and the lycopene amount of the tomato fruit were obtained as an average value of values measured in four portions on an equator in which a diameter of the tomato fruit harvested in Examples and Comparative Examples is the largest, which are four portions on which a line connecting two portions is orthogonal to a line connecting other two portions, using a fruit vegetable quality evaluation device Fruit Selector (model: K-BA800, manufactured by Kubota Co., Ltd.). The calculation results were summarized in Table 2.

In addition, the lycopene amount obtained by using Fruit Selector was specifically measured as follows. First, a calibration curve was created by correlating the lycopene amount of the tomato fruit measured by high-speed liquid chromatography (HPLC) with the lycopene amount measured by Fruit Selector. Next, the lycopene amount measured using Fruit Selector was converted into the lycopene amount measured by HPLC based on the calibration curve, and the lycopene amount was adopted.

[Table 1]

| | Regarding irradiation light | | | | Lightperiod temperature (°C) | Darkperiod temperature (°C) | Carbon dioxide concentration (ppm) | Fertilizer feeding method | EC value of liquid fertilizer (ds/m) | Sum of number of flower clusters and fruit clusters (X) | Length from planting surface to growth point of main branch (L) | L/X (cm/piece) |
| | Type of light | Light intensity (μmol/m²·s) | Light-dark cycle | Light composition | | | | | | | | |
|---|---|---|---|---|---|---|---|---|---|---|---|---|
| Example 1 | Artificial light | 250 | 1.4 | Light composition 1 | 25 | 17 | 400 | Flooded hydroponic cultivation | 2.0 | 5 | 90 | 180 |
| Example 2 | Artificial light | 500 | 2.0 | Light composition 2 | 25 | 19 | 1000 | Drop type hydroponic cultivation | 3.5 | 4 | 45 | 11.3 |
| Example 3 | Artificial light | 800 | 1.4 | Light composition 3 | 25 | 17 | 400 | Flooded hydroponic cultivation | 1.5 | 3 | 19 | 6.3 |
| Example 4 | Artificial light | 200 | 1.4 | Light composition 4 | 25 | 17 | 800 | Flooded hydroponic cultivation | 1.5 | 3 | 88 | 29.3 |
| Example 5 | Artificial light | 300 | 2.0 | Light composition 4 | 30 | 17 | 1000 | Drop type hydroponic cultivation | 3.5 | 4 | 95 | 238 |
| Example 6 | Artificial light | 500 | 2.0 | Light composition 5 | 27 | 19 | 1000 | Drop type hydroponic cultivation | Irregular type | 5 | 65 | 130 |

| | Regarding irradiation light | | | | Light period temperature (°C) | Dark period temperature (°C) | Carbon dioxide concentration (ppm) | Fertilizer feeding method | EC value of liquid fertilizer (ds/m) | Sum of number of flower clusters and fruit clusters (X) | Length from planting surface to growth point of main branch (L) | L/X (cm/piece) |
|---|---|---|---|---|---|---|---|---|---|---|---|---|
| | Type of light | Light intensity ($\mu$mol/m²·s) | Light-dark cycle | Light composition | | | | | | | | |
| Example 7 | Artificial light | 500 | 2.0 | Light composition 4 | 25 | 19 | 1000 | Drop type hydroponic cultivation | 6.0 | 3 | 65 | 21.7 |
| Comparative Example 1 | Sunlight | - | - | - | 35 | 18 | 400 | Drop type hydroponic cultivation | 2.0 | 3 | 106 | 35.3 |
| Comparative Example 2 | Artificial light | 900 | 1.4 | Light composition 6 | 25 | 17 | 400 | Flooded hydroponic cultivation | 2.0 | 3 | 15 | 5.0 |

EP 4 245 129 A1

[Table 2]

| | Evaluation | | |
|---|---|---|---|
| | Quality evaluation | | Number of harvests (piece) |
| | Lycopene amount (mg/100 g) | Brix sugar content (% by mass) | |
| Example 1 | 15 | 4.2 | 15 |
| Example 2 | 17 | 4.8 | 16 |
| Example 3 | 18 | 4.0 | 3 |
| Example 4 | 15 | 3.9 | 9 |
| Example 5 | 8.5 | 4.5 | 20 |
| Example 6 | 19 | 100 | 20 |
| Example 7 | 16 | 7.2 | 6 |
| Comparative Example 1 | 5.6 | 5.6 | 12 |
| Comparative Example 2 | - | - | 0 |

From the results of the Examples, it can be recognized that according to the tomato plant according to the present disclosure, the space utilization efficiency in cultivation and the quality of the harvested tomato fruits are improved.

The disclosure of JP2020-190528 filed November 16, 2020 is incorporated herein by reference in its entirety. All documents, patent applications, and technical standards described in the present specification shall be incorporated by reference to the same extent as specifically and individually stated that individual documents, patent applications, and technical standards are incorporated by reference.

**Claims**

1.    A tomato plant that satisfies Formula (1):

$$6.0 \leq L/X \leq 30.0 \ ... \ (1)$$

wherein in the formula, in a case where the tomato plant does not have a side branch, L represents a length in cm unit from a planting surface to a growth point of a main branch, and X represents a sum of the number of flower clusters and fruit clusters of the main branch, and

in a case where the tomato plant has a side branch, L represents a length in cm unit from the planting surface to the growth point of one main branch or side branch, and X represents a sum of the number of flower clusters and fruit clusters of the one main branch or side branch,

where, X is an integer of 2 or more.

2.    The tomato plant according to claim 1,
wherein in Formula (1), L is 100 or less.

3.    The tomato plant according to claim 1 or 2,
wherein in Formula (1), X is an integer of 10 or less.

4.    The tomato plant according to any one of claims 1 to 3, which satisfies Formula (2):

$$11.0 \leq L/X \leq 22.0 \ ... \ (2)$$

wherein in the formula, in a case where the tomato plant does not have a side branch, L represents a length in cm unit from a planting surface to a growth point of a main branch, and X represents a sum of the number of flower clusters and fruit clusters of the main branch, and

in a case where the tomato plant has a side branch, L represents a length in cm unit from the planting surface to the growth point of one main branch or side branch, and X represents a sum of the number of flower clusters and fruit clusters of the one main branch or side branch,

where, X is an integer of 2 or more.

5. A tomato fruit which is harvested from the tomato plant according to any one of claims 1 to 4 and comprises 10 mg or more of lycopene per 100 g by weight.

6. The tomato fruit according to claim 5,
wherein a Brix sugar content is 7% by mass or more.

7. A method of cultivating a tomato plant, at least comprising: a step of irradiating a ground part of a first tomato plant with artificial light having a light intensity of 200 $\mu$mol/m$^2$·s to 800 $\mu$mol/m$^2$·s,

wherein a second tomato plant that satisfies Formula (1) is cultivated from the first tomato plant:

$$6.0 \leq L/X \leq 30.0 \ ... \ (1)$$

wherein in the formula, in a case where the second tomato plant does not have a side branch, L represents a length in cm unit from a planting surface to a growth point of a main branch, and X represents a sum of flower clusters and fruit clusters of the main branch, and

in a case where the second tomato plant has a side branch, L represents a length in cm unit from the planting surface to the growth point of one main branch or side branch, and X represents a sum of the number of flower clusters and fruit clusters of the one main branch or side branch,

where, X is an integer of 2 or more.

8. The method of cultivating a tomato plant according to claim 7,
wherein irradiation of the ground part of the first tomato plant with the artificial light is performed from a side surface direction of the first tomato plant.

9. The method of cultivating a tomato plant according to claim 7 or 8,
wherein a light-dark cycle of the artificial light with which the ground part of the first tomato plant is irradiated is controlled, and a ratio of a light period time to a dark period time is 1 to 3.

10. The method of cultivating a tomato plant according to any one of claims 7 to 9,
wherein a cultivation temperature of the first tomato plant in a light period is 23°C to 33°C, and the cultivation temperature of the first tomato plant in a dark period is 15°C to 22°C.

11. The method of cultivating a tomato plant according to any one of claims 7 to 10,
wherein a carbon dioxide concentration of the first tomato plant in a cultivation environment is 300 ppm or more.

12. The method of cultivating a tomato plant according to any one of claims 7 to 11,
wherein a cultivation device comprising one or more selected from the group consisting of a light source for irradiating the ground part of the first tomato plant with the artificial light from at least one of an upper surface direction or a side surface direction, a hydroponic cultivation mechanism, and a temperature/humidity control mechanism is used.

FIG. 1

# FIG. 2

# FIG. 3

## INTERNATIONAL SEARCH REPORT

| International application No. |
| --- |
| **PCT/JP2021/037933** |

**A. CLASSIFICATION OF SUBJECT MATTER**

*A01G 22/05*(2018.01)i; *A01G 7/00*(2006.01)i; *A01G 7/02*(2006.01)i; *A01G 31/00*(2018.01)i
FI: A01G22/05 Z; A01G7/00 601C; A01G7/02; A01G31/00 612

According to International Patent Classification (IPC) or to both national classification and IPC

**B. FIELDS SEARCHED**

Minimum documentation searched (classification system followed by classification symbols)

A01G22/05; A01G7/00; A01G7/02; A01G31/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus (JDreamIII); JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | 直立 1 本整枝法, 野菜園芸大百科　トマト, 第 2 版，第 2 刷, 社団法人農山漁村文化協会, 15 April 2005, pp. 455 - 458 "(1) Features of training method", "(3) Precautions for use", "1 Importance of controlling the initial growth", fig. 2, 4 | 1-4 |
| Y | "(1) Features of training method", "(3) Precautions for use", "1 Importance of controlling the initial growth", fig. 2, 4, non-official translation (Vertical single-staking methods. Encyclopedia of vegetable gardening: Tomato. 2nd edition. 2nd printing. RURAL CULTURE ASSOCIATION JAPAN.) | 7-12 |
| X | JP 2017-104060 A (JUJO PAPER CO LTD) 15 June 2017 (2017-06-15) paragraphs [0012], [0019] | 5-6 |
| Y | paragraph [0028] | 11 |
| X | US 2018/0199527 A1 (AMMERLAAN, Arnoldus Cornelis Josef) 19 July 2018 (2018-07-19) paragraphs [0001], [0012], [0014] | 5-6 |
| Y | JP 2012-70727 A (HAMAMATSU PHOTONICS KK) 12 April 2012 (2012-04-12) paragraphs [0026]-[0027] | 7-12 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 December 2021** | **28 December 2021** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2021/037933** |

### C. DOCUMENTS CONSIDERED TO BE RELEVANT

| Category* | Citation of document, with indication, where appropriate, of the relevant passages | Relevant to claim No. |
| --- | --- | --- |
| Y | JP 2011-50288 A (FUJIKIN INC) 17 March 2011 (2011-03-17)<br>paragraphs [0019]-[0022], fig. 1-2 | 8, 12 |
| A | 鈴木　克己, トマトの低段密植・多収栽培, 農耕と園藝, 01 October 2008, vol. 63, no. 10, pp. 39-43<br>entire text, all drawings, (SUZUKI, Katsumi. Agriculturist and Horticulturist.), non-official translation (Tomato with low node-order pinching and high-density planting and high-yielding cultivation) | 1-12 |

Form PCT/ISA/210 (second sheet) (January 2015)

**INTERNATIONAL SEARCH REPORT**
Information on patent family members

International application No.

**PCT/JP2021/037933**

| Patent document cited in search report | | | Publication date (day/month/year) | Patent family member(s) | | | Publication date (day/month/year) |
|---|---|---|---|---|---|---|---|
| JP | 2017-104060 | A | 15 June 2017 | (Family: none) | | | |
| US | 2018/0199527 | A1 | 19 July 2018 | WO | 2017/007328 | A1 | |
| | | | | NL | 2015116 | B1 | |
| JP | 2012-70727 | A | 12 April 2012 | (Family: none) | | | |
| JP | 2011-50288 | A | 17 March 2011 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5330162 B
- JP 2016202072 A
- JP 2020190528 A